# EUROPEAN PATENT SPECIFICATION

(11) **EP 3 367 993 B1**
(45) Date of publication and mention of the grant of the patent: **19.11.2025**
(21) Application number: 15804286.1
(22) Date of filing: 20.11.2015
(51) Int. Cl.: A61K 8/25, A61Q 11/00, A61K 8/73

(54) **ORAL CARE COMPOSITION COMPRISING CHITOSAN AND SILICA**
MUNDPFLEGEZUSAMMENSETZUNG ENTHALTEN CHITOSAN UND KIESELSÄURE
COMPOSITION À SOIN ORAL CONTENANT CHITOSANE ET SILICE

(43) Date of publication of application: 05.09.2018
(73) Proprietor: Colgate-Palmolive Company, New York, NY 10022 (US)
(72) Inventor: FONTERS, Jessie, 1092 Belmont-sur-Lausanne (CH); LÖTSCHER, Jeannine, 4107, Ettingen (CH); BRUNELLA, André, 4143 Dornach (CH)
(74) Representative: Wibbelmann, Jobst
(86) International application number: PCT/US2015/061929
(87) International publication number: WO 2017/086998

(56) References cited:
- WO-A1-2004/045446
- WO-A1-2014/202232
- CN-A- 103 006 455
- CN-A- 103 110 536
- CN-A- 104 288 466
- CN-A- 104 825 542
- US-A1- 2008 267 891
- US-A1- 2013 323 186

## Description

### BACKGROUND

Dentin is a portion of the tooth internal to the enamel and cementum that has a radially striated appearance owing to a large number of fine canals or tubules known as the dentinal tubules. Tubules run from the pulp cavity to the periphery of the dentin and are generally about two microns in diameter at their base and somewhat narrower at their periphery. Tubules are not usually exposed to the environment in the oral cavity, as they are usually covered by enamel or cementum. The cementum in turn is often covered by the gums.

It is believed that in certain cases tooth sensitivity may be due to the exposure of dentin, which covers the nerve, either through loss of the enamel layer or recession of the gums. The dentin contains a large number of microtubules that run from the outside of the tooth to the nerve in the center of the tooth. When the dentin is exposed, the microtubules can transmit stimuli, e.g., from changes in temperature or from certain foods (acidic or sweet) to the nerve, causing the tooth or teeth to perceive pain. The pain usually subsides after a short period of time.

It is commonly understood that partially or fully exposed tubules can lead to tooth sensitivity, an irritating and painful condition. In this theory, recession of the gum line exposes cementum to erosion. The eroded cementum in turn exposes the hollow dentinal tubules. The exposed tubules cause nerves within the tooth to be affected excessively by external oral stimuli because material and energy transfer between the exterior and interior of the tooth is accelerated through the tubules. Common environmental stimuli, such as heat, cold, chemicals and physical and mechanical pressure or stimuli, such as brushing, are able to irritate the nerve through the open dentin tubules and thereby create pain. The pain of sensitive teeth appears to result from these stimuli, which apparently cause fluid movements in the dentinal tubules that activate pulpal nerve endings.

Conventionally, two approaches have been taken to treat or ameliorate tooth sensitivity. Under one approach, the chemical environment proximal to the nerve is altered by application of various agents, such that the nerve is not stimulated, or not stimulated as greatly. Known agents useful in this chemical approach may include, for example, potassium salts (such as potassium nitrate, potassium bicarbonate, potassium chloride), strontium salts, zinc salts, and chloride salts.

The second approach involves the mechanical shield of the nerve by, e.g., blocking of the dentinal tubules wholly or partially with "tubule blocking agents." Agents that have been disclosed in the prior art include, for example, cationic alumina, clays, water-soluble or water-swellable polyelectrolytes, oxalates, amorphous calcium phosphate, hydroxyapatite, maleic acid copolymers and polyethylene particles.

However, both the chemical and the mechanical approaches, because they require the incorporation of one or more additional materials to the dentifrice, may result in formulation difficulties, either technical or related to increased costs. For this reason there is a need in the art for a dentifrice that, upon use, prevents or reduces tooth sensitivity yet is not associated with significant processing or formulation disadvantages. Thus, despite the many products on the market and the many efforts to address this problem over the years, there still remains a need for safe and effective oral care products to address the problem of dental hypersensitivity. US 2008/0267891 A1 discloses an oral care composition that reduces and/or eliminates the perception of tooth sensitivity, wherein the composition includes an adherent material and particles having an average particle size of about 8 microns or less, wherein the particles are present in the composition in an amount of about 5% by weight. US 2013/0323186 A1 discloses a dentifrice, such as toothpaste, containing chitosan or pharmaceutically acceptable acid addition salt thereof with fluoride ions, for use against erosive tooth demineralization, and kits containing chitosan or pharmaceutically acceptable acid addition salt thereof with fluoride ions, wherein one of the two active agents is comprised in a dentifrice, are described, wherein the dentifrice in the form of toothpaste may furthermore comprise dissolved tin, in particular stannous ions. WO 2004/045446 A1 discloses a solid, controlled-dissolution composition for the oral cavity that adheres to hard dental surfaces, such as natural teeth and the surrounding soft tissue, and which creates a physical barrier to painful stimuli associated with dentinal hypersensitivity.

### BRIEF SUMMARY

Disclosed herein are oral care compositions for treating tooth hypersensitivity comprising chitosan and at least one small particle silica as further defined in the claims. Also disclosed herein are oral care compositions for treating tooth hypersensitivity comprising chitosan and at least one small particle silica, wherein the oral care composition has an acidic pH. The oral care composition for treating tooth hypersensitivity comprises chitosan, at least one small particle silica, and at least one non-ionic cellulose ether, wherein the oral care composition has an acidic pH as further defined in the claims. In certain exemplary embodiments, the at least one non-ionic cellulose polymer is hydroxyethyl cellulose.

The oral care compositions for treating tooth hypersensitivity have an acidic pH as further defined in the claims, such as a pH less than about 6, for example less than about 5.5, less than about 5, less than about 4.5, or about 4.5. In certain exemplary embodiments, the pH of the oral care composition may be adjusted with an acid, such as hydrochloric acid or an organic acid such as malic acid.

The oral care compositions disclosed herein have a flow reduction greater than 45% as further defined in the claims, such as greater than about 50%, greater than about 55%, greater than about 60%, greater than about 65%, or greater than about 70%, when measured using a hydraulic conductance test.

The chitosan is present in an amount ranging from 0.3% to 1% as further defined in the claims. In various embodiments of the oral care compositions disclosed herein, the at least one small particle silica is present in an amount ranging from about 5% to about 10%. The small particle silica has a median particle size ranging from 2 µm to 4 µm as further defined in the claims.

In certain exemplary embodiments, the hydroxyethyl cellulose may be present in the oral care composition disclosed herein in an amount ranging from about 0.1% to about 5%. In certain embodiments, the oral care compositions disclosed herein may further comprise at least one fluoride ion source, such as an amine fluoride. In certain embodiments the at least one fluoride ion source, such as the amine fluoride, may be present in an amount of about 1.8% by weight.

Methods of treating or preventing tooth hypersensitivity can be comprising applying to a tooth surface an oral care composition comprising chitosan and at least one small particle silica, wherein the oral care composition has an acidic pH. The methods of treating or preventing tooth hypersensitivity can further comprise having or causing a flow reduction greater than about 45%, when measured using a hydraulic conductance test.

Further disclosed herein are compositions for use in methods for reducing dental sensitivity as further defined in the claims. Further disclosed herein are compositions for use in methods of occluding a dentin tubule within the surface of a tooth comprising applying to a tooth surface an oral care composition comprising chitosan and at least one small particle silica, wherein the oral care composition has an acidic pH as further defined in the claims. The methods of occluding a dentin tubule within the surface of a tooth further comprise causing a flow reduction greater than 45%, when measured using a hydraulic conductance test as further defined in the claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention which is defined in the claims.

### DETAILED DESCRIPTION

The following description of the preferred embodiments is merely exemplary in nature and is in no way intended to limit the invention, its application, or uses which is defined in the claims.

As used throughout, ranges are used as shorthand for describing each and every value that is within the range. In the event of a conflict in a definition in the present disclosure and that of a cited reference, the present disclosure controls.

As used herein, the term "one or more of" with respect to a listing of items such as, for example, A and B, means A alone, B alone, or A and B. The term "at least one of" is used to mean one or more of the listed items can be selected.

Unless otherwise specified, all percentages and amounts expressed herein and elsewhere in the specification should be understood to refer to percentages by weight. The amounts given are based on the active weight of the material.

Disclosed herein are oral care compositions for treating and/or preventing tooth hypersensitivity comprising chitosan and at least one small particle silica as further defined in the claims. The compositions can be used in methods of treating and/or preventing tooth sensitivity comprising applying to the tooth surface an oral care composition comprising chitosan and at least one small particle silica.

The oral care composition may comprise a small particle silica having a median particle size ranging from 2 µm to 4 µm as further defined in the claims. The oral compositions within the scope of the invention can also include at least one small particle silica having a median particle size that is no greater than the average diameter of a human dentin tubule, such that one or more particles are capable of becoming lodged within the tubule, thereby effecting a reduction or elimination of perceived tooth sensitivity. In certain embodiments, the at least one small particle silica may be chosen from ZEODENT^{®}, SIDENT^{®}, SORBOSIL^{®}, TIXOSIL^{®}, and combinations thereof. In certain embodiments, the at least one small particle silica is chosen from SORBOSIL^{®} AC43.

It is understood that methods for measuring particle size are known in the art, and based on the disclosure set forth herein and methods known in the art, one of ordinary skill in the art will understand how to calculate median particle size, mean particle size, and/or particle size distribution of silica particles as disclosed herein.

The small particle silica as defined in the claims has a particle size characterized by the parameters of a median particle size of 2 µm to 4 µm, optionally a d₁₀ of about 0.5 µm to about 2 µm, and a d₉₀ of about 5 µm to about 10 µm. As used herein, d₁₀ refers to particles having a diameter that is 10% of the threshold of the sampled population (i.e., 10% of the population is equal to or smaller than the d₁₀ value), and d₉₀ refers to particles having a diameter that is 90% of the threshold of the sampled population (i.e., 90% of the population is equal to or smaller than the d₉₀ value).

In other embodiments disclosed herein, at least a portion of the small particle silica in the composition has a d₅₀ of 3.95 µm (i.e., 50% of the population of the silica particles is equal to or smaller than the d₅₀ value). SORBOSIL^{®} AC43 silica has a d₅₀ of 3.95 µm. By way of non-limiting example, the d₅₀ may be measured using particle size measuring techniques such as the Malvern Mastersizer technique.

In the Malvern Mastersizer technique, particle size distribution is measured using a Malvern Particle Size Analyzer, such as a Model Mastersizer 2000 (or comparable model) (Malvern Instruments, Inc., Southborough, Mass.), wherein a helium-neon gas laser beam is projected through a transparent cell which contains silica, such as, for example, silica hydrogel particles suspended in an aqueous solution. Light rays that strike the particles are scattered through angles that are inversely proportional to the particle size. The photodetector arrant measures the quantity of light at several predetermined angles. Electrical signals proportional to the measured light flux values are then processed by a microcomputer system, against a scatter pattern predicted from theoretical particles as defined by the refractive indices of the sample and aqueous dispersant to determine the particle size distribution of the silica hydrogel, for example. It will be understood that other methods of measuring particle size are known in the art, and based on the disclosure set forth herein, the skilled artisan will understand how to calculate median particle size, mean particle size, and/or particle size distribution of silica particles of the present invention.

Described herein are oral care compositions wherein the small particle silica particles may be initially present in the composition having the desired particle size, or alternatively, the silica particles may be initially present in the composition at a larger size, so long as the structure of the particles is such that it fractures or breaks into the desired particle size upon application of mechanical force by, for example, a toothbrush, when brushing or applying the composition. In certain embodiments, the at least one small particle silica in the oral care compositions disclosed herein may be present in an amount ranging from about 1% to about 20%, such as about 1% to about 10%, about 5% to about 10%, or about 5%.

The oral care compositions disclosed herein comprise, in addition to the at least one small particle silica, further chitosan or chitosan derivatives as further defined in the claims. Chitosan is a linear polysaccharide that is composed of randomly distributed β-(1-4)-linked D-glucosamines (deacetylated units) and N-acetyl-D-glucosamines (acetylated units). Chitosan is derived from chitin, which is the second most abundant occurring polymer in nature and is a component of the cell walls of fungi, the exoskeletons of arthropods such as crustaceans and insects, and the beak and pen of some cephalopods such as squid. In certain embodiments, the oral care compositions disclosed herein may comprise either chitosan of crustacean origin or chitosan of fungal origin. For example, shrimp chitosan or Aspergillus chitosan may be used in certain embodiments. Chitosan preparations may include, for example, glucan. Chitosan may be synthesized from chitin by the deacetylation of chitin using sodium hydroxide. Chitosan is considered a cationic polymer that is water-soluble and has bioadhesive properties, such that it readily binds to negatively charged surfaces. The chitosan and/or chitosan derivatives in the oral care compositions disclosed herein is present in an amount ranging from 0.3% to 1% as defined in the claims, such as about 0.5% to 1%, about 0.5%, or 1%.

The oral care composition further comprises a non-ionic cellulose ether. In certain embodiments, the non-ionic cellulose ether may be hydroxyethyl cellulose. The at least one polymer material may be any known or to be developed in the art, such as the hydroxyethyl cellulose sold under the brand name TYLOSE^{®}. The at least one polymer material may be, for example, any homopolymers or copolymers (hereinafter referred to collectively as a "polymers") that adhere to the surface of a tooth. Such polymers may include cellulose polymers, for example one or more hydroxyalkyl cellulose polymers, such as hydroxypropylmethyl cellulose (HPMC), hydroxyethylpropyl cellulose (HEPC), hydroxybutylmethyl cellulose (HBMC), carboxymethyl cellulose (CMC), hydroxyl propyl cellulose, hydroxyethyl cellulose, methyl cellulose, and mixtures thereof. In one embodiment the at least one polymer material comprises a mixture of cellulose materials, for example a mixture of two hydroxyalkyl cellulose materials having different molecular weight. In certain embodiments, the at least one polymer, such as a non-ionic cellulose ether, may be present in the composition in an amount ranging from about 0.1% to about 5%, such as about 1.5% to about 2.5%.

The oral care compositions disclosed herein are acidic as further defined in the claims, and in certain embodiments may have a pH of less than about 7, such as less than about 6.5, less than about 6, less than 5.5, less than about 5, or less than about 4.5. In certain embodiments, the oral care compositions disclosed herein have a pH of about 4.5. In certain exemplary embodiments, the pH may range from about 4.0 to about 5.0, such as about 4.0 to about 4.5, about 4.25 to about 4.75, or about 4.5 to about 5.0. As one of ordinary skill in the art would recognize, the pH of the compositions disclosed here may be adjusted by addition of an acid, such a hydrochloric acid or organic acids known in the art, such as, for example, malic acid.

In certain exemplary embodiments, the composition may be substantially free of silica dimethyl silylate. In certain embodiments, the composition may be substantially free of polyethylene. As used herein, the term "substantially free of" means about 0% by weight or an amount that is so low as to not have a reasonable chemical effect on the composition.

In certain embodiments, the oral care composition may further comprise at least one fluoride ion source, such as a soluble fluoride salt. A wide variety of fluoride ion sources can be in the embodiments of the compositions disclosed herein. Examples of suitable fluoride ion sources include, but are not limited to, stannous fluoride, sodium fluoride, potassium fluoride, sodium monofluorophosphate, sodium fluorosilicate, ammonium fluorosilicate, amine fluorides such as olaflur (N,N,N'-tris(2-hydroxyethyl)-N'-octadecyl-1,3-diaminopropane dihydrofluoride), ammonium fluoride, and combinations thereof. In certain embodiments, the oral care composition comprises amine fluoride. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, such as about 500 to about 2000 ppm, about 1000 to about 1600 ppm, or about 1450 ppm, depending on the particular application. Fluoride ion sources may be present in the oral compositions disclosed herein in an amount ranging from about 0.01% to about 15%, such as about 0.03% to about 10%, or about 0.1% to about 5% by weight of the composition. Weights of fluoride salts to provide the appropriate level of fluoride ion will obviously vary based on the weight of the counter ion in the salt.

The oral compositions may further comprise, in addition to the at least one small particle silica, one or more abrasive particulates. Any abrasive particulates may be used and may be selected from sodium bicarbonate, calcium phosphate (e.g., dicalcium phosphate dihydrate), calcium sulfate, precipitated calcium carbonate, silica (e.g., hydrated silica), iron oxide, aluminum oxide, perlite, plastic particles, e.g., polyethylene, and combinations thereof. In particular, the abrasive may be selected from a calcium phosphate (e.g., dicalcium phosphate dihydrate), calcium sulfate, precipitated calcium carbonate, silica (e.g., hydrated silica), calcium pyrophosphate and combinations thereof. Any type of silica may be used, such as precipitated silicas or silica gels. In certain embodiments, the oral care composition comprises at least one silica that has a particle size and an amount and distribution in the oral care composition such that the at least one silica has a dual function, and may function not only as a dentin tubule-occluding particulate but also as an abrasive particulate.

In some embodiments, the oral care composition may include any other therapeutic, cosmetic, and/or aesthetic materials as may be desired. Examples include non-silica desensitizing agents (e.g. a nitrate salt, an arginine ester, a bicarbonate salt, potassium nitrate, an arginine-bicarbonate-phytate complex, potassium citrate, and arginine, among others), a chemical whitening agent (such as a peroxide releasing compound), an opaque whitening agent (such as hydroxyapatite) and an anticalculus agent. Other options for inclusion in the oral care composition of the invention include triclosan; stannous ion agents; chlorhexidine; alexidine; hexetidine; sanguinarine; benzalkonium chloride; salicylanilide; domiphen bromide; cetylpyridinium chloride (CPC); tetradecylpyridinium chloride (TPC); N-tetradecyl-4-ethylpyridinium chloride (TDEPC); octenidine; delmopinol; octapinol; nisin; zinc ion agents; copper ion agents; essential oils; furanones; bacteriocins, ethyl lauroyl arginate, extracts of magnolia, a metal ion source, arginine bicarbonate, honokiol, magonol, ursolic acid, ursic acid, morin, extract of sea buckthorn, an enzyme, a Camellia extract, a flavonoid, a flavan, halogenated diphenyl ether, creatine, and propolis.

The composition according to the present invention may comprise an antimicrobial agent which may be selected from halogenated diphenyl ether (triclosan), herbal extracts or essential oils (e.g., rosemary extract, thymol, menthol, eucalyptol, methyl salicylate), bisguanide antiseptics (e.g., chlorhexidine, alexidine, or octenidine), phenolic antiseptics, hexetidine, povidone iodine, delmopinol, salifluor, sanguinarine, propolis, oxygenating agents (e.g., hydrogen peroxide, buffered sodium peroxyborate, or peroxycarbonate), cetyl pyridinium chloride, magnolia extract, magnolol, honokiol, butyl magnolol, propyl honokiol, substituted aminoalkans (e.g., N,N,N'-tris(2-hydroxyethyl)-N'-octadecyl-1,3-diaminopropane), and mixtures thereof. Anti-attachment agents such as Solrol also can be included, as well as plaque dispersing agents such as enzymes (papain, glucoamylase, etc.).

The composition according to the present invention may also comprise one or more further agents typically selected from anti-plaque agents, whitening agents, antibacterial agents, cleaning agents, flavoring agents, sweetening agents, adhesion agents, surfactants, foam modulators, abrasives, pH modifying agents, humectants, mouth feel agents, colorants, abrasive, tartar control (anticalculus) agent, saliva stimulating agents, nutrients, and combinations thereof. Various components that may be added to the composition include, for example, at least one sweetening agent such as saccharin or sodium saccharin, alcohols such as ethanol, as well as glycerine, sorbitol, propylene glycol, polyethylene glycols, Poloxomer polymers such as POLOXOMER 407, PLURONIC F108, (both available from BASF Corporation), alkyl polyglycoside (APG), polysorbate, PEG40, castor oil, menthol, and the like.

Flavorants among those useful herein include any material or mixture of materials operable to enhance the taste of the composition. Any orally acceptable natural or synthetic flavorant can be used, such as flavoring oils, flavoring aldehydes, esters, alcohols, similar materials, and combinations thereof. Flavorants include vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants and mixtures thereof. Also encompassed within flavorants herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, [alpha]-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methane glycerol acetal (MGA), and mixtures thereof. One or more flavorants are optionally present in a total amount of about 0.01% to about 5%, optionally in various embodiments from about 0.05 to about 2%, from about 0.1% to about 2.5%, and from about 0.1% to about 0.5%.

Sweetening agents among those useful herein include dextrose, polydextrose, sucrose, maltose, dextrin, dried invert sugar, mannose, xylose, ribose, fructose, levulose, galactose, corn syrup, partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones, and mixtures thereof.

Colorants among those useful herein include pigments, dyes, lakes and agents imparting a particular luster or reflectivity such as pearling agents. In various embodiments, colorants are operable to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including FD&C dyes and pigments, talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride and mixtures thereof. One or more colorants are optionally present in a total amount of about 0.001% to about 20%, for example about 0.01% to about 10% or about 0.1% to about 5%. In certain embodiments, the titanium dioxide may be present in the oral composition in an amount ranging from about 0.1% to about 5%, such as about 0.9% to about 2%, or about 1%.

The compositions of the present invention optionally comprise a tartar control (anticalculus) agent. Tartar control agents among those useful herein include salts of any of these agents, for example their alkali metal and ammonium salts: phosphates and polyphosphates (for example pyrophosphates), polyaminopropanesulfonic acid (AMPS), polyolefin sulfonates polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof. In certain aspects disclosed herein, oral care compositions comprising chitosan and at least one small particle silica can desensitize a tooth.

The compositions disclosed herein may optionally comprise a saliva stimulating agent useful, for example, in amelioration of dry mouth. Any orally acceptable saliva stimulating agent can be used including without limitation food acids such as citric, lactic, malic, succinic, ascorbic, adipic, fumaric and tartaric acids, and mixtures thereof. One or more saliva stimulating agents are optionally present in saliva stimulating effective total amount.

The compositions of the present invention optionally comprise at least one nutrient. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophane L-lysine, methionine, threonine, levocarnitine and L-carnitine), lipotropics (such as choline, inositol, betaine, and linoleic acid), and mixtures thereof.

The oral care compositions disclosed herein may be formulated into any delivery form that permits contact of the formulation comprising the chitosan and the at least one small particle silica to the tooth surface. For example, the compositions may be formulated into a mouth rinse, a gel, a lozenge (dissolvable or chewable), a spray, a gum, and a film (wholly or partially dissolvable, or indissoluble). In certain exemplary embodiments, the oral care composition is a gel, such as a toothpaste gel. The composition may comprise any conventional excipients or carriers, although these will vary depending on the dosage form or means of dosage selected.

In other aspects, such compositions may provide tooth desensitization that is superior to conventional desensitizing dentifrices. By way of a non-limiting example, dentifrices disclosed herein comprising chitosan and at least one small particle silica may provide tooth desensitization by providing a greater desensitization than a conventional dentifrice or a conventional desensitizing dentifrice, by providing desensitization more rapidly than a conventional dentifrice or a conventional desensitizing dentifrice, or by a combination of greater desensitization and more rapid desensitization, among other effects. In an embodiment, an oral care composition comprising chitosan and at least one small particle silica as disclosed herein and as further defined in the claims provides desensitization and/or superior desensitization in the absence of any other desensitizing agent. In another embodiment, an oral care composition comprising chitosan and at least one small particle silica as disclosed herein and as further defined in the claims provides desensitization and/or superior desensitization, and may contain one or more additional desensitizing agents, such as additional silica particles, as described elsewhere herein.

Methods of use and/or application of a desensitizing composition can be comprising chitosan and at least one small particle silica. Such a composition may be applied to the tooth via conventional brushing techniques (e.g., use of a toothbrush). A composition comprising chitosan and at least one small particle silica as disclosed herein may be applied to the tooth via a method other than conventional brushing techniques. Other methods of application include manual application (e.g., applying a composition to a tooth using one or more fingers, rubbing onto the tooth surface, rubbing in a circular motion, etc.), or application using any known dental appliance or applicator. It will be understood, based on the disclosure set forth herein, that any method of smearing a composition onto a tooth, optionally using varying degrees of physical pressure, is encompassed in the scope defined by the claims.

Desensitization of a tooth may be measured by any technique set forth herein, or any technique known to the skilled artisan. Application of the composition to the tooth surface results in the introduction of the composition into one or more dentin tubules. The composition is applied to the teeth by any method set forth herein or known in the art.

Hydraulic Conductance testing may be performed to evaluate the degree of occlusion taking place on a dentin segment attached to a sensor that measures the displacement of water or other liquid over time. The occlusion efficacy is related to a decrease in the hydraulic conductance, or water permeability of dentin segments after treatment with dentifrice. The baseline may be measured on a dentin segment previously etched to have the maximum open tubules and higher permeability ("0% occlusion"). A decrease in the hydraulic conductance after treatment with a dentifrice indicates the occlusion effect, calculated in the percentage of flow reduction.

Hydraulic conductance may be measured by any method known in the art. Fluid flow rate, i.e., hydraulic conductance is measured as defined in the claim. In certain embodiments, dentin segments, such as human dentin segments, are cut from extracted teeth, such as extracted molars, etched, mounted, and connected to a sensor to measure hydraulic conductance. In certain embodiments, the dentin discs may be from about 0.4 mm to about 0.8 mm thick, such as about .45 mm to about .48 mm thick. Segments may then be treated with various formulations, and then rinsed.

In certain exemplary embodiments, the apparatus may include a tube attached to a flow sensor. An air bubble may be injected into the tube. By measuring the displacement of the bubble as a function of time, fluid flow through the dentin disc can be measured. Fluid flow is equivalent to the dentin permeability. In certain exemplary embodiments, the dentin disk may be affixed to a holder and placed in a measurement cell, wherein the pressure is standardized, for example at about 100 cm H₂O. As the water flows through the dentin disk, the flow may then be measured as a function of the change in pressure in the measurement cell, wherein the flow in the flow measurement cell is from the pulpal to the occlusal side of the dentin disk.

Blocks may then be rinsed again and incubated in water or a buffer solution, such as a phosphate buffered saline (PBS, which may approximate intra-oral conditions by simulating artificial saliva) before the next treatment. In certain embodiments, the compositions disclosed herein result in or cause a flow reduction after application of the composition from a measured baseline of greater than about 45%, such as greater than about 40%, greater than about 55%, greater than about 60%, greater than about 65%, greater than about 68% or greater than about 70%.

In certain embodiments, the dentin segments may be challenged, for example challenged with an acid solution, such as a citric acid solution or a cola solution, and hydraulic conductance may be measured again, where the percentage of reduction in fluid flow after each treatment and acid challenge may be measured.

### EXAMPLES

### Example 1

A control toothpaste formulation (Control 1) was prepared that did not comprise chitosan and also did not comprise a small particle silica. A second control toothpaste formulation (Control 2) was prepared that likewise did not comprise chitosan but did comprise a small particle silica. The formulations of both Control 1 and Control 2 are shown below in Table 1. Additionally, several sample toothpaste formulations (Formulations A-D) were prepared comprising varying quantities of ingredients, including both chitosan and at least one small particle silica, as show below in Table 2.

**Table 1 - Control Formulations**

| **Ingredient** | **Control 1** | **Control 2** |
|---|---|---|
| Chitosan | -- | -- |
| Hydroxyethyl cellulose | 2.5% | 2.5% |
| Amine Fluoride | 1.84% | 1.84% |
| Small particle silica | -- | 5% |
| Hydrated silica | 11% | 10% |
| PEG-40 Hydrogenated Castor Oil | 2% | -- |
| HCl 10% | | 0.76% |
| Water, fragrance, sweetener, colorant | QS | QS |
| pH | 4.74 | 4.29 |

**Table 2 - Sample Formulations A-D**

| **Ingredient** | **Formula A** | **Formula B** | **Formula C** | **Formula D** |
|---|---|---|---|---|
| Shrimp chitosan | 0.5% | -- | -- | -- |
| Aspergillus chitosan | -- | 1% | 1% | 1% |
| Hydroxyethyl cellulose | 1.5% | 2.2% | 2.2% | 2.2% |
| Amine Fluoride | 1.84% | 1.84% | 1.84% | 1.84% |
| Small particle silica | 5% | 5% | 5% | 5% |
| Hydrated silica | 10% | 10% | 10% | 10% |
| HCl 10% | 1% | 2.37% | 1.10% | -- |
| Malic acid | -- | -- | -- | 0.32% |
| Water, fragrance, sweetener , colorant | QS | QS | QS | QS |
| pH | 4.48 | 4.23 | 4.61 | 4.47 |

### Example 2

The formulations prepared in Example 1 were evaluated for flow reduction over several runs in a hydraulic conductance model. To evaluate the occlusion efficacy of the dentifrice formulas, dentin disks were prepared from human molars. After extraction, human molars were stored in a thymol/ethanol (5%) solution. Then they were disinfected in a 10% H2O2 solution for 24-48h. Following to this procedure, the teeth are stored again in a thymol/ethanol (5%) solution until usage.

The human molars were sliced to dentin disks of 450-480 µm thickness and washed for two minutes in a 2% citric acid solution, followed by a 60 second ultrasonic bath in order to open the tubules on the dentin surface. Then, the dentin disks were stored in 70% ethanol for 24-48 h prior to the respective experiment to prevent swelling of native proteins in the dentin disk. The dentin disks were once more stored for 2 min in 6% citric acid, followed by a 60 s ultrasonic bath. The disks were then rinsed for 30 seconds with a phosphate buffered saline (PBS, pH 7) solution. Next, the baseline hydraulic conductance of the disks was measured before exposure to any dentifrice formulation. The hydraulic conductance was measured over approximately 30 minutes, with a maximum flow of 300 µL, and the time recorded every 20 µL. The pressure was standardized at 100 cm H₂0.

Each disk was then brushed for one minute with 300 uL of a 1:2 slurry of the dentifrice formula (2 parts water: 1 part toothpaste), then stored for one minute in the dentifrice slurry, and then rinsed with 5 ml PBS. After the brushing, storing, and rinsing cycle was repeated 3 times, the disks were again measured for hydraulic conductance over approximately 30 minutes and a minimum of 80 µL, with the time recording every 20 µL. The procedure was repeated for all dentifrice formulas, including both Control 1 and Control 2, as well as sample Formulations A-D. Multiple series of testing were performed. The results of the hydraulic conductance studies are shown below in Table 3.

**Table 3 - Percent Flow Reduction for Control and Sample Formulations after Toothpaste Application**

| **Formula** | **Test Series 3-9** | **Standard Deviation** |
|---|---|---|
| **Control 1** | 23.1% (n=24) | 31.6% |
| **Control 2** | 41.2% (n=22) | 20.8% |
| **Formula A** | 61.7% (n=43) | 20.4% |
| **Formula B** | 51.5% (n=35) | 18.0% |
| **Formula C** | 68.2% (n=23) | 18.2% |
| **Formula D** | 53.0% (n=23) | 19.4% |

As shown above in Table 3, formulations comprising a combination of chitosan and at least one small particle silica exhibited a reduction in flow over the control formulations that did not comprise chitosan and a small particle silica. The data provided herein evidences that the addition of chitosan to a toothpaste formulation comprising at least one small particle silica may increase flow reduction and consequently shows a good occlusion of the dentin tubules.

### Example 3

After the toothpaste application (i.e., application of Control 1, Control 2, and Formulations A-D) described in Example 2, some of the disks (i.e., Control 2, Formulation A and Formulation B) were next exposed to 1 min citric acid challenge (using both a 1% citric acid solution and a 6% citric acid solution at pH 5) and then rinsed with PBS. The disks were again measured for hydraulic conductance after the acid challenges in the same manner as before, over approximately 30 minutes and a minimum of 80 µL, with the time recorded every 20 µL.

The results are shown below in Table 4 (showing the results of the 1% citric acid challenge) and Table 5 (showing the results of the 6% citric acid challenge). As can be seen from Tables 4 and 5 below, the effect of the flow reduction remained even after the dentin disks were challenged with both 1% and 6% citric acid.

**Table 4 - % Flow Reduction Before and After 1 Minute, 1% Citric Acid Challenge**

| **Formulation** | **Flow reduction (mean % ± SD) after toothpaste challenge** | **Flow reduction (mean % ± SD) after acid challenge** |
|---|---|---|
| Control 2 | 49.5±25.9 (n=11) | 47.2±29.0 (n=11) |
| Formula A | 74.2±11.6 (n=11) | 75.8±10.7 (n=11) |
| Formula B | 60.2±13.6 (n=12) | 57.4±11.2 (n=12) |

**Table 5 - % Flow Reduction Before and After 1 Minute, 6% Citric Acid Challenge**

| **Formulation** | **Flow reduction (mean % ±** SD**) after toothpaste challenge** | **Flow reduction (mean % ±** SD**) after acid challenge** |
|---|---|---|
| Control 1 | 9.7±35.3 (n=12) | -2.9±33.0 (n=11) |
| Control 2 | 32.8±9.0 (n=11) | 25.4±15.3 (n=11) |
| Form. A | 45.7±20.5 (n=12) | 54.5±15.0 (n=11) |
| Form. B | 49.2±14.4 (n=12) | 47.9±26.0 (n=12) |

## Claims

1. An oral care composition for reducing hypersensitivity in a tooth comprising:
chitosan in an amount ranging from 0.3% to 1%, by weight based on the total weight of the composition;
a small particle silica having a median particle size ranging from 2 µm to 4 µm; and
a non-ionic cellulose ether, wherein the oral care composition has an acidic pH,
wherein said oral care composition causes a flow reduction through human dentin greater than 45%, when measured using a hydraulic conductance test.

2. The oral care composition according to claim 1, wherein the at least one non-ionic
cellulose ether is hydroxyethyl cellulose.

3. The oral care composition according to claim 1 or 2, wherein the pH of oral care composition is less than 5.

4. The oral care composition according to claim 1 or 2, wherein the pH of the oral care composition ranges from 4.0 to 5.0.

5. The oral care composition according to any of the preceding claims, wherein the pH of the oral care composition is adjusted with an acid chosen from hydrochloric acid or malic acid.

6. The oral care composition according to any of the preceding claims, having a flow reduction through human dentin greater than 50%, when measured using a hydraulic conductance test.

7. The oral care composition according to any of the preceding claims, further comprising amine fluoride.

8. The oral care composition according to any of the preceding claims, wherein the small particle silica has a d₉₀ of 5 µm to 10 µm.

9. The oral care composition according to any of the preceding claims, wherein the small particle silica is present in an amount ranging from 5% to 10%, by weight based on the total weight of the composition.

10. An oral care composition comprising
chitosan in an amount ranging from 0.3% to 1%, by weight based on the total weight of the composition;
a small particle silica having a median particle size ranging from 2 µm to 4 µm; and
a non-ionic cellulose ether, wherein the oral care composition has an acidic pH for use in a method for reducing dental sensitivity comprising:
applying to a tooth surface the oral care composition;
wherein said oral care composition causes a flow reduction through human dentin greater than 45%, when measured using a hydraulic conductance test.

11. The oral care composition for use in a method according to claim 10, wherein the oral care composition causes a flow reduction through human dentin greater than 50%, when measured using a hydraulic conductance test.

12. An oral care composition comprising
chitosan in an amount ranging from 0.3% to 1%, by weight based on the total weight of the composition;
a small particle silica having a median particle size ranging from 2 µm to 4 µm; and
a non-ionic cellulose ether, wherein the oral care composition has an acidic pH;
for use in a method of occluding dentin tubules;
wherein said oral care composition causes a flow reduction through the dentin tubules greater than 45%, when measured using a hydraulic conductance test.

## Patentansprüche

1. Mundpflegezusammensetzung zum Verringern von Überempfindlichkeit in einem Zahn, umfassend:
Chitosan in einer Menge im Bereich von 0,3 % bis 1 %, bezogen auf das Gesamtgewicht der Zusammensetzung;
ein kleinpartikuläres Silica mit einer mittleren Partikelgröße im Bereich von 2 µm bis 4 µm; und
einen nicht-ionischen Celluloseether, wobei die Mundpflegezusammensetzung einen sauren pH-Wert aufweist,
wobei die Mundpflegezusammensetzung eine Verringerung des Durchflusses durch menschliches Dentin von mehr als 45 % bewirkt, wenn es mit einem hydraulischen Leitfähigkeitstest gemessen wird.

2. Mundpflegezusammensetzung nach Anspruch 1, wobei der mindestens eine nicht-ionische Celluloseether Hydroxyethylcellulose ist.

3. Mundpflegezusammensetzung nach Anspruch 1 oder 2, wobei der pH-Wert der Mundpflegezusammensetzung weniger als 5 beträgt.

4. Mundpflegezusammensetzung nach Anspruch 1 oder 2, wobei der pH-Wert der Mundpflegezusammensetzung im Bereich von 4,0 bis 5,0 liegt.

5. Mundpflegezusammensetzung nach einem beliebigen der vorhergehenden Ansprüche, wobei der pH-Wert der Mundpflegezusammensetzung mit einer Säure, ausgewählt aus Salzsäure oder Äpfelsäure, eingestellt wird.

6. Mundpflegezusammensetzung nach einem beliebigen der vorhergehenden Ansprüche, die eine Verringerung des Durchflusses durch menschliches Dentin von mehr als 50 % aufweist, wenn es mit einem hydraulischen Leitfähigkeitstest gemessen wird.

7. Mundpflegezusammensetzung nach einem beliebigen der vorhergehenden Ansprüche, die weiterhin Aminfluorid umfasst.

8. Mundpflegezusammensetzung nach einem beliebigen der vorhergehenden Ansprüche, wobei das kleinpartikuläre Silica einen d₉₀ von 5 µm bis 10 µm aufweist.

9. Mundpflegezusammensetzung nach einem beliebigen der vorhergehenden Ansprüche, wobei das kleinpartikuläre Silica in einer Menge von 5 % bis 10 %, bezogen auf das Gesamtgewicht der Zusammensetzung, vorhanden ist.

10. Mundpflegezusammensetzung, umfassend
Chitosan in einer Menge im Bereich von 0,3 % bis 1 %, bezogen auf das Gesamtgewicht der Zusammensetzung;
ein kleinpartikuläres Silica mit einer mittleren Partikelgröße im Bereich von 2 µm bis 4 µm; und
einen nicht-ionischen Celluloseether, wobei die Mundpflegezusammensetzung einen sauren pH-Wert aufweist, zur Verwendung in einem Verfahren zum Verringern der Zahnempfindlichkeit, umfassend:
Auftragen der Mundpflegezusammensetzung auf eine Zahnoberfläche;
wobei die Mundpflegezusammensetzung eine Verringerung des Durchflusses durch menschliches Dentin von mehr als 45 % bewirkt, wenn es mit einem hydraulischen Leitfähigkeitstest gemessen wird.

11. Mundpflegezusammensetzung zur Verwendung in einem Verfahren nach Anspruch 10, wobei die Mundpflegezusammensetzung eine Verringerung des Durchflusses durch menschliches Dentin von mehr als 50 % bewirkt, wenn es mit einem hydraulischen Leitfähigkeitstest gemessen wird.

12. Mundpflegezusammensetzung, umfassend
Chitosan in einer Menge im Bereich von 0,3 % bis 1 %, bezogen auf das Gesamtgewicht der Zusammensetzung;
ein kleinpartikuläres Silica mit einer mittleren Partikelgröße von 2 µm bis 4 µm; und
einen nicht-ionischen Celluloseether, wobei die Mundpflegezusammensetzung einen sauren pH-Wert aufweist
zur Verwendung in einem Verfahren zum Verschließen von Dentintubuli;
wobei die Mundpflegezusammensetzung eine Verringerung des Durchflusses durch die Dentintubuli von mehr als 45 % bewirkt, wenn es mit einem hydraulischen Leitfähigkeitstest gemessen wird.

## Revendications

1. Composition de soin bucco-dentaire destinée à réduire l'hypersensibilité d'une dent, comprenant :
du chitosane en une quantité allant de 0,3 % à 1 %, en poids par rapport au poids total de la composition ;
une silice à petites particules ayant une taille de particule médiane allant de 2 µm à 4 µm ; et
un éther de cellulose non ionique, dans laquelle la composition de soin bucco-dentaire a un pH acide,
dans laquelle ladite composition de soin bucco-dentaire provoque une réduction de flux à travers la dentine humaine supérieure à 45 %, lorsqu'il est mesuré à l'aide d'un test de conductance hydraulique.

2. Composition de soin bucco-dentaire selon la revendication 1, dans laquelle ledit au moins un éther de cellulose non ionique est de l'hydroxyéthylcellulose.

3. Composition de soin bucco-dentaire selon la revendication 1 ou 2, dans laquelle le pH de la composition de soin bucco-dentaire est inférieur à 5.

4. Composition de soin bucco-dentaire selon la revendication 1 ou 2, dans laquelle le pH de la composition de soin bucco-dentaire est compris entre 4,0 et 5,0.

5. Composition de soin bucco-dentaire selon l'une quelconque des revendications précédentes, dans laquelle le pH de la composition de soin bucco-dentaire est ajusté à l'aide d'un acide choisi parmi l'acide chlorhydrique ou l'acide malique.

6. Composition de soin bucco-dentaire selon l'une quelconque des revendications précédentes, présentant une réduction de flux à travers la dentine humaine supérieure à 50 %, lorsqu'il est mesuré à l'aide d'un test de conductance hydraulique.

7. Composition de soin bucco-dentaire selon l'une quelconque des revendications précédentes, comprenant en outre du fluorure d'amine.

8. Composition de soin bucco-dentaire selon l'une quelconque des revendications précédentes, dans laquelle la silice à petites particules a un d₉₀ de 5 µm à 10 µm.

9. Composition de soin bucco-dentaire selon l'une quelconque des revendications précédentes, dans laquelle la silice à petites particules est présente en une quantité allant de 5 % à 10 %, en poids par rapport au poids total de la composition.

10. Composition de soin bucco-dentaire comprenant
du chitosane en une quantité allant de 0,3 % à 1 %, en poids par rapport au poids total de la composition ;
une silice à petites particules ayant une taille de particule médiane allant de 2 µm à 4 µm ; et
un éther de cellulose non ionique, dans laquelle la composition de soin bucco-dentaire a un pH acide pour une utilisation dans un procédé de réduction de la sensibilité dentaire comprenant :
l'application sur une surface dentaire de la composition de soin bucco-dentaire ;
dans laquelle ladite composition de soin bucco-dentaire provoque une réduction de flux à travers la dentine humaine supérieure à 45 %, lorsqu'il est mesuré à l'aide d'un test de conductance hydraulique.

11. Composition de soin bucco-dentaire pour une utilisation dans un procédé selon la revendication 10, dans laquelle la composition de soin bucco-dentaire provoque une réduction de flux à travers la dentine humaine supérieure à 50 %, lorsqu'il est mesuré à l'aide d'un test de conductance hydraulique.

12. Composition de soin bucco-dentaire comprenant
du chitosane en une quantité allant de 0,3 % à 1 %, en poids par rapport au poids total de la composition ;
une silice à petites particules ayant une taille de particule médiane allant de 2 µm à 4 µm ; et
un éther de cellulose non ionique, dans laquelle la composition de soin bucco-dentaire a un pH acide ;
pour une utilisation dans un procédé d'occlusion des tubules dentinaires ;
dans laquelle ladite composition de soin bucco-dentaire provoque une réduction de flux à travers les tubules dentinaires supérieure à 45 %, lorsqu'il est mesuré à l'aide d'un test de conductance hydraulique.
